# EUROPEAN PATENT SPECIFICATION

(11) **EP 3 689 246 B1**
(45) Date of publication and mention of the grant of the patent: **25.02.2026**
(21) Application number: 19195411.4
(22) Date of filing: 04.09.2019
(51) Int. Cl.: A61B 8/00, A61B 8/08

(54) **ULTRASOUND DIAGNOSIS APPARATUS AND METHOD OF OPERATING THE SAME**
ULTRASCHALLDIAGNOSEVORRICHTUNG UND VERFAHREN ZUM BETRIEB DAVON
APPAREIL DE DIAGNOSTIC PAR ULTRASONS ET SON PROCÉDÉ DE FONCTIONNEMENT

(30) Priority: 29.01.2019 KR 20190011307
(43) Date of publication of application: 05.08.2020
(73) Proprietor: Samsung Medison Co., Ltd., Hongcheon-gun, Gangwon-do, 25108 (KR)
(72) Inventor: YIM, Yuri, 13530 Gyeonggi-do (KR); PARK, Seoklai, 13530 Gyeonggi-do (KR); LEE, Youngseok, 13530 Gyeonggi-do (KR)
(74) Representative: Arnold & Siedsma

(56) References cited:
- MOEHRING M A ET AL: "Power M-mode Doppler (PMD) for observing cerebral blood flow and tracking emboli", ULTRASOUND IN MEDICINE AND BIOLOGY, NEW YORK, NY, US, vol. 28, no. 1, 1 January 2002 (2002-01-01), pages 49 - 57, XP004342776, ISSN: 0301-5629, DOI: 10.1016/S0301-5629(01)00486-0
- EVA CAVERO ET AL: "SPIHT-Based Echocardiogram Compression: Clinical Evaluation and Recommendations of Use", IEEE JOURNAL OF BIOMEDICAL AND HEALTH INFORMATICS, IEEE, PISCATAWAY, NJ, USA, vol. 17, no. 1, 1 January 2013 (2013-01-01), pages 103 - 112, XP011494175, ISSN: 2168-2194, DOI: 10.1109/TITB.2012.2227336

## Description

### BACKGROUND

### 1. Field

The disclosure relates to ultrasound diagnosis apparatuses and methods of operating the same, and more particularly, to ultrasound diagnosis apparatuses and methods of operating the same, which are capable of providing a motion (M)-mode image simultaneously with a Doppler sound.

### 2. Description of Related Art

Recently, in the medical field, various types of medical imaging apparatuses have been widely used to visualize and acquire information about living tissue of a human body for early diagnosis or surgery with regard to various diseases. Representative examples of these medical imaging apparatuses may include an ultrasound diagnosis apparatus, a computed tomography (CT) apparatus, and a magnetic resonance imaging (MRI) apparatus.

Ultrasound diagnosis apparatuses transmit ultrasound signals generated by transducers of a probe to an object and receive information of echo signals reflected from the object, thereby obtaining an image of an internal part of the object. In particular, ultrasound diagnosis apparatuses are used for medical purposes including observing an internal area of an object, detecting foreign substances, and assessing injuries. Such ultrasound diagnosis apparatuses exhibit high stability, display images in real-time, and are safe due to lack of radiation exposure compared to diagnostic X-ray apparatuses. Therefore, the ultrasound diagnosis apparatuses have been widely used together with other types of diagnostic imaging apparatuses.

The publication M. A. Moehring et al. "Power M-mode Doppler (PMD) for observing cerebral blood flow and tracking emboli", Ultrasound in Medicine an Biology, vol. 28, no. 1, pages 49-57, XP004342776, discloses an ultrasound diagnosis apparatus comprising:
a. a memory storing one or more instructions;
b. a processor configured to execute the one or more instructions to:
   - acquire, based on echo signals received from an object, motion (M) mode data with respect to the object with a first period and Doppler data with respect to the object with a second period (the first and second periods being identical),
   - generate an M mode image based on the M mode data,
   - control a display to display the M mode image,
   - generate a Doppler sound based on the Doppler data, and
   - control a speaker to output the Doppler sound; and
c. a probe configured to transmit an ultrasound signal to the object and receive the echo signals from the object;
d. wherein the processor is further configured execute the one or more instructions to:
   - control the probe to transmit with the first period a first ultrasound signal corresponding to a first ultrasound pulse according to an M-mode pulse repetition frequency to the object and acquire the M mode data based on a first echo signal corresponding to the first ultrasound signal, and
   - control the probe to transmit with the second period a second ultrasound signal corresponding to a second ultrasound pulse according to a pulsed wave mode pulse repetition frequency to the object and acquire the Doppler data based on a second echo signal corresponding to the second ultrasound signal (the first and second ultrasound pulses being identical);
e. wherein the second period is determined based on the first period.

When a fetal heart is examined in a Doppler mode of an ultrasound diagnosis apparatus during early pregnancy, stability problems may occur due to energy applied to the fetal heart. Thus, several academic societies in the field of sonography have recommended limiting the use of a Doppler ultrasound for examination of a fetus in the early stage (the first trimester) of pregnancy.

In addition, when the fetal heart is examined only in an ultrasound M-mode, a Doppler sound of the fetal heart cannot be provided.

### SUMMARY

Provided are ultrasound diagnosis apparatuses and methods of operating the same, which are capable of minimizing an effect on the heart of a fetus (in particular, in the early stage of pregnancy) while providing a Doppler sound of the fetal heart. The invention is defined in the claims.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a block diagram illustrating an ultrasound diagnosis apparatus according to an exemplary embodiment;
FIGS. 2A, 2B, and 2C are diagrams respectively illustrating an ultrasound diagnosis apparatus according to an exemplary embodiment;
FIG. 3 is a flowchart of a method of operating an ultrasound diagnosis apparatus, according to an embodiment;
FIG. 4 is a reference diagram for explaining a method, performed by an ultrasound diagnosis apparatus, of acquiring motion (M)-mode data and Doppler data, according to an embodiment;
FIG. 5 is a reference diagram for explaining a method, performed by an ultrasound diagnosis apparatus, of adjusting a quality of a Doppler sound, according to an embodiment;
FIG. 6 illustrates an example of a screen displayed by an ultrasound diagnosis apparatus, according to an embodiment;
FIGS. 7A through 7D are reference diagrams for explaining a method, performed by an ultrasound diagnosis apparatus, of generating and outputting a Doppler sound while operating in an M-mode, according to an embodiment;
FIG. 8 illustrates an example of a user interface (UI) capable of adjusting a quality of a Doppler sound output by an ultrasound diagnosis apparatus, according to an embodiment;
FIG. 9 is a flowchart of a method of operating an ultrasound diagnosis apparatus, according to an embodiment;
FIG. 10 illustrates an example of a screen displayed by an ultrasound diagnosis apparatus, according to an embodiment; and
FIG. 11 is a block diagram of a configuration of an ultrasound diagnosis apparatus according to an embodiment.

### DETAILED DESCRIPTION

Certain exemplary embodiments are described in greater detail below with reference to the accompanying drawings.

**In** the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements. Expressions such as "at least one of," when preceding a list of elements, modify the entire list of elements and do not modify the individual elements of the list.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

Hereinafter, embodiments of the disclosure will be described in detail with reference to the accompanying drawings.

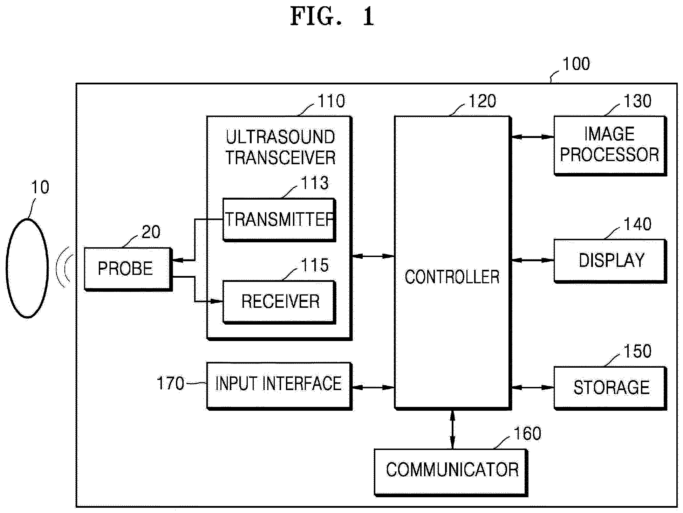

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus, which is portable, moveable, mobile, or hand-held. Examples of the portable-type ultrasound diagnosis apparatus may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control an ultrasound receiver 115 to generate ultrasound data by converting reception signals received from the probe 20 from analog to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet PCs, wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, a button, a keypad, a mouse, a trackball, a jog switch, a knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

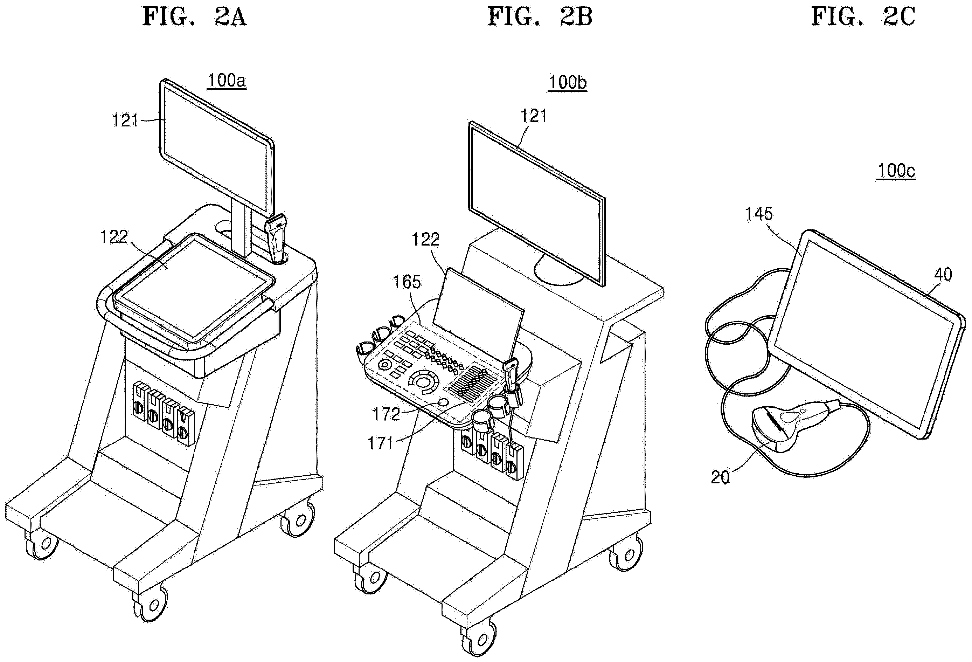

An example of the ultrasound diagnosis apparatus 100 according to the present exemplary embodiment is described below with reference to FIGS. 2A, 2B, and 2C.

FIGS. 2A, 2B, and 2C are diagrams illustrating an ultrasound diagnosis apparatus according to an exemplary embodiment.

Referring to FIGS. 2A and 2B, the ultrasound diagnosis apparatus 100a or 100b may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100a or 100b. The main display 121 and the sub-display 122 may provide graphical user interfaces (GUIs), thereby receiving user's inputs of data to control the ultrasound diagnosis apparatus 100a or 100b. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100a or 100b may control the display of the ultrasound image on the main display 121 by using the input control data.

Referring to FIG. 2B, the ultrasound diagnosis apparatus 100b may include a control panel 165. The control panel 165 may include buttons, trackballs, jog switches, or knobs and may receive data to control the ultrasound diagnosis apparatus 100b from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100b may keep displaying a frame image at that time point.

The buttons, trackballs, jog switches, and knobs included in the control panel 165 may be provided as a GUI to the main display 121 or the sub-display 122.

Referring to FIG. 2C, an ultrasound diagnosis apparatus 100c may include a portable device. An example of the portable ultrasound diagnosis apparatus may include smart phones including probes and applications, laptop computers, PDAs, or tablet PCs, but an exemplary embodiment is not limited thereto.

The ultrasound diagnosis apparatus 100c may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100c, and a GUI.

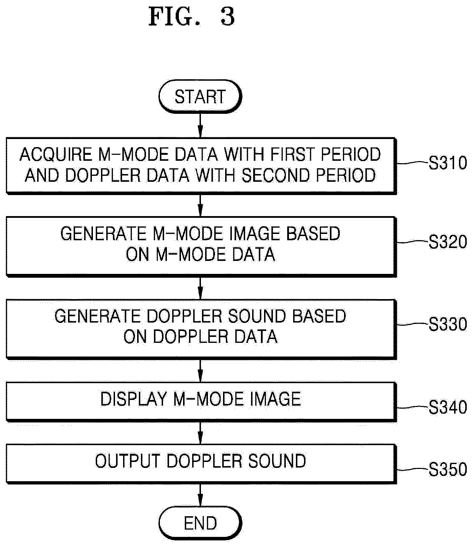

FIG. 3 is a flowchart of a method of operating an ultrasound diagnosis apparatus, according to an embodiment.

According to an embodiment, the ultrasound diagnosis apparatus 100 may transmit ultrasound signals to an object and acquire ultrasound data based on echo signals received from the object. For example, the ultrasound diagnosis apparatus 100 may acquire brightness (B)-mode data with respect to the object based on echo signals. The ultrasound diagnosis apparatus 100 may then generate a B-mode ultrasound image based on the B-mode data and display the generated B-mode ultrasound image. The ultrasound diagnosis apparatus 100 may extract B-mode components from ultrasound data and generate a B-mode ultrasound image indicating signal intensities as brightness values based on the extracted B-mode components.

Furthermore, the ultrasound diagnosis apparatus 100 may set one of a plurality of scan lines included in the B-mode ultrasound image as an M line. The ultrasound diagnosis apparatus 100 may also set a region located on the M line as a region of interest (ROI).

The ultrasound diagnosis apparatus 100 may acquire M-mode data corresponding to an M line with a first period and Doppler data corresponding to an ROI with a second period (S310).

The ultrasound diagnosis apparatus 100 may acquire M-mode data indicating signal intensities as brightness values over time based on ultrasound data corresponding to an M line.

Furthermore, the ultrasound diagnosis apparatus 100 may extract Doppler components from ultrasound data corresponding to an ROI and acquire Doppler data based on the extracted Doppler components. In this case, a second period with which the Doppler data is acquired may be determined based on the first period with which the M-mode data is acquired. For example, the ultrasound diagnosis apparatus 100 may set the first and second periods such that the Doppler data is acquired once each time the M-mode data is acquired n times (n is an integer value), but embodiments are not limited thereto.

Referring back to FIG. 3, the ultrasound diagnosis apparatus 100 may generate an M-mode image based on the M-mode data (S320).

M-mode data may represent motion information of the object corresponding to the M line with respect to time, and the ultrasound diagnosis apparatus 100 may generate an M-mode image based on the M-mode data.

Furthermore, the ultrasound diagnosis apparatus 100 may generate a Doppler sound based on the Doppler data (S330).

The ultrasound diagnosis apparatus 100 may interpolate unacquired Doppler data corresponding to an interval during which Doppler data is not acquired by using discontinuously acquired Doppler data. The ultrasound diagnosis apparatus 100 may generate a Doppler sound by using the acquired and interpolated Doppler data.

The ultrasound diagnosis apparatus 100 may display the M-mode image (S340) and output the Doppler sound (S350).

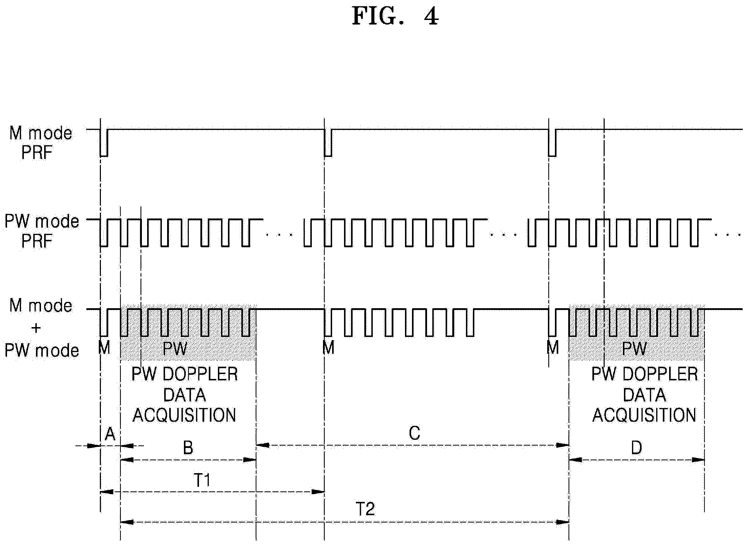

FIG. 4 is a reference diagram for explaining a method, performed by an ultrasound diagnosis apparatus, of acquiring M-mode data and Doppler data, according to an embodiment.

Referring to FIG. 4, the ultrasound diagnosis apparatus 100 may generate a first ultrasound pulse according to an M-mode pulse repetition frequency (PRF). The ultrasound diagnosis apparatus 100 may transmit a first ultrasound signal corresponding to the first ultrasound pulse to an object and receive a first echo signal corresponding to the first ultrasound signal. The ultrasound diagnosis apparatus 100 may acquire M-mode data based on the first echo signal and generate an M-mode image based on the M-mode data.

Furthermore, the ultrasound diagnosis apparatus 100 may generate a second ultrasound pulse according to a pulsed wave (PW) mode PRF. The ultrasound diagnosis apparatus 100 may transmit a second ultrasound signal corresponding to the second ultrasound pulse to the object and receive a second echo signal corresponding to the second ultrasound signal. The ultrasound diagnosis apparatus 100 may acquire Doppler data based on the second echo signal and generate a Doppler sound based on the Doppler data.

In addition, according to an embodiment, the ultrasound diagnosis apparatus 100 may acquire the M-mode data together with the Doppler data by generating the first ultrasound pulse alternately with the second ultrasound pulse. For example, as shown in FIG. 4, during a first interval A, the ultrasound diagnosis apparatus 100 may generate a first ultrasound pulse and transmit the first ultrasound pulse to the object to thereby acquire M-mode data. During a second interval B, the ultrasound diagnosis apparatus 100 may generate a second ultrasound pulse and transmit the second ultrasound pulse to the object to thereby acquire Doppler data.

A period of repetition_of an M-mode data acquisition interval during which M-mode data is acquired (an interval during which a first ultrasound pulse sequence is generated) may be a first period T1. Furthermore, a period of repetition of a Doppler data acquisition interval during which Doppler data is acquired (an interval during which a second ultrasound pulse sequence is generated) may be a second period T2. In this case, the second period T2 may be determined based on the first period T1. For example, as shown in FIG. 4, the second period T2 may be double the first period T1. In this case, the ultrasound diagnosis apparatus 100 may acquire Doppler data once while acquiring M-mode data twice, but embodiments are not limited thereto.

In addition, the ultrasound diagnosis apparatus 100 may generate Doppler data corresponding to an interval during which Doppler data is not acquired by using the acquired Doppler data. For example, when pieces of Doppler data are respectively acquired during second and fourth intervals B and D and Doppler data is not acquired during a third interval C, the ultrasound diagnosis apparatus 100 may interpolate unacquired Doppler data corresponding to the third interval C by using the pieces of Doppler data acquired during the second and fourth intervals B and D.

The ultrasound diagnosis apparatus 100 may generate an M-mode image based on the M-mode data and generate a Doppler sound based on the acquired and interpolated Doppler data to be output.

When the ultrasound diagnosis apparatus 100 operates in M-mode plus Doppler mode, the amount of energy applied to the object (e.g., a fetus) is reduced compared to when operating only in a Doppler mode. Thus, according to an embodiment, the ultrasound diagnosis apparatus 100 may perform an ultrasound scan on a fetal heart in M-mode plus Doppler mode, thereby minimizing the amount of energy applied to the fetal heart and providing a Doppler sound together with an M-mode image.

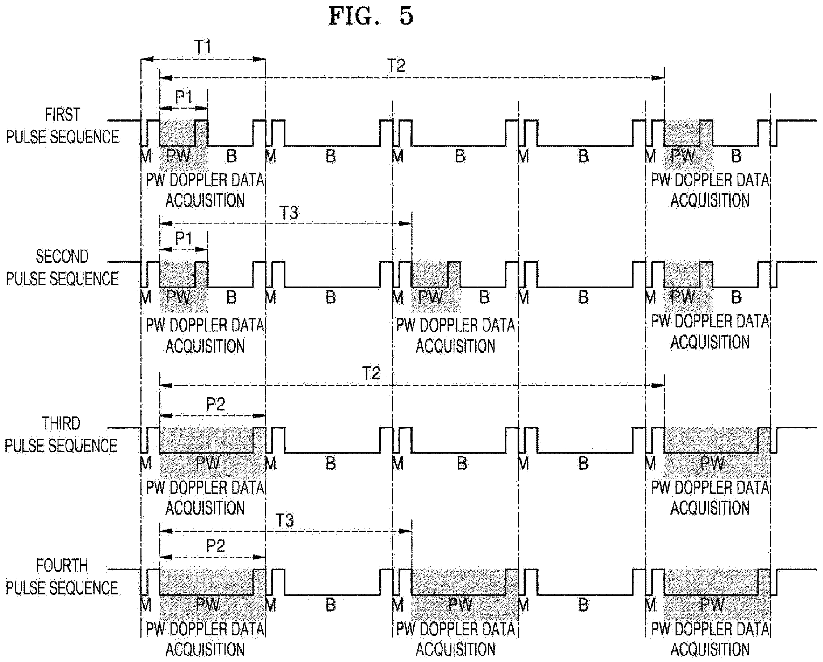

FIG. 5 is a reference diagram for explaining a method, performed by an ultrasound diagnosis apparatus, of adjusting a quality of a Doppler sound, according to an embodiment.

According to an embodiment, the ultrasound diagnosis apparatus 100 may control a quality of a Doppler sound by adjusting a Doppler data acquisition period.

Referring to FIG. 5, the ultrasound diagnosis apparatus 100 may acquire M-mode data, Doppler data, and B-mode data. For example, M-mode data and Doppler data may be respectively acquired with a first period T1 and a second period T2 while B-mode data may be acquired without any specific period. Furthermore, the B-mode data may be acquired in an interval during which the M-mode data and the Doppler data are not acquired.

During an M-mode data acquisition interval, the ultrasound diagnosis apparatus 100 may generate a first ultrasound pulse to transmit a first ultrasound signal corresponding to the first ultrasound pulse to an object and receive a first echo signal from the object. Furthermore, during a Doppler data acquisition interval, the ultrasound diagnosis apparatus 100 may generate a second ultrasound pulse to transmit a second ultrasound signal corresponding to the second ultrasound pulse to the object and receive a second echo signal therefrom. In addition, during a B-mode data acquisition interval, the ultrasound diagnosis apparatus 100 may generate a third ultrasound pulse according to a B-mode PRF to transmit a third ultrasound signal corresponding to the third ultrasound pulse to the object and receive a third echo signal therefrom.

In this case, the ultrasound diagnosis apparatus 100 may adjust a quality of a Doppler sound generated based on Doppler data by adjusting a period or length of a Doppler data acquisition interval.

For example, a first ultrasound pulse sequence (an M-mode pulse sequence) included in a first pulse sequence of FIG. 5 may have a first period T1, and a second ultrasound pulse sequence (a PW mode pulse sequence) included in the first pulse sequence may have a second period T2 and a first length P1. The ultrasound diagnosis apparatus 100 may acquire Doppler data by using the first pulse sequence and generate a Doppler sound with a first quality based on the acquired Doppler data.

Furthermore, the ultrasound diagnosis apparatus 100 may set a period of the second ultrasound pulse sequence to a third period T3 shorter than the second period T2 as in a second pulse sequence of FIG. 5 to thereby generate a Doppler sound with a second quality higher than the first quality.

Alternatively, the ultrasound diagnosis apparatus 100 may set, as in a third pulse sequence of FIG. 5, a period of the second ultrasound pulse sequence to the second period T2 and a length thereof to a second length P2 greater than the first length P1 to thereby generate a Doppler sound with a third quality higher than the first quality.

Alternatively, the ultrasound diagnosis apparatus 100 may set, as in a fourth pulse sequence of FIG. 5, a period of the second ultrasound pulse sequence to the third period T3 and a length thereof to the second length P2 to thereby generate a Doppler sound with a fourth quality higher than the first through third qualities.

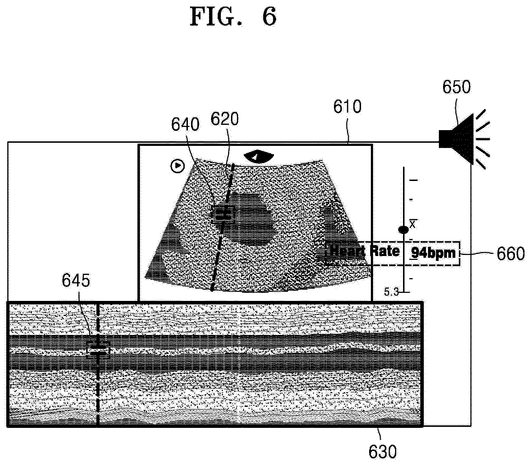

FIG. 6 illustrates an example of a screen displayed by the ultrasound diagnosis apparatus 100, according to an embodiment.

Referring to FIG. 6, the ultrasound diagnosis apparatus 100 may generate a B-mode image 610 based on B-mode data with respect to an object and display the B-mode image 610 on the display 140.

The ultrasound diagnosis apparatus 100 may display an M line 620 superimposed on one of a plurality of scan lines in the B-mode image 610 and change a position of the M line 620 based on a user input. The ultrasound diagnosis apparatus 100 may generate an M-mode image 630 based on M-mode data corresponding to the M line 620 and display the M-mode image 630 on the display 140.

Furthermore, the ultrasound diagnosis apparatus 100 may generate a Doppler sound 650 based on Doppler data corresponding to an ROI located on the M line 620 and output the Doppler sound 650 via a speaker. Furthermore, an ROI 645 may be indicated on the M-mode image 630.

Thus, according to an embodiment, the ultrasound diagnosis apparatus 100 may display the B-mode image 610 and the M-mode image 630 and simultaneously output the Doppler sound 650. Furthermore, when the object is a heart (in particular, a fetal heart), the ultrasound diagnosis apparatus 100 may detect a heart rate 660 from M-mode data and display the detected heart rate 660 on the display 140. Furthermore, according to an embodiment, the ultrasound diagnosis apparatus 100 may compare the detected heart rate with a preset reference value to determine whether there is an abnormality in the detected heart rate and display a determining result on the display 140.

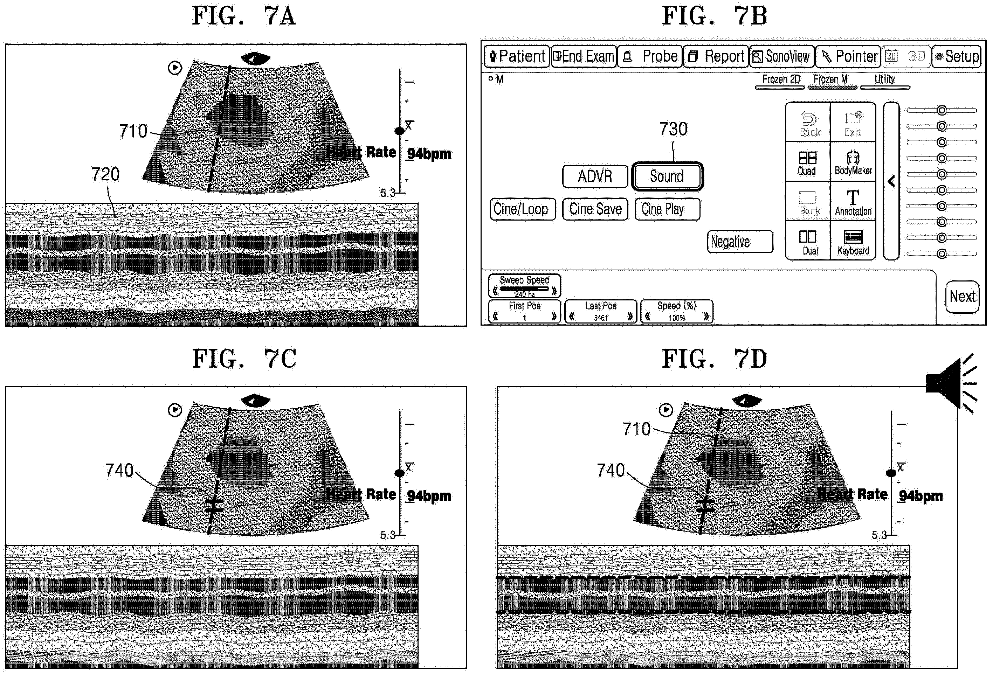

FIGS. 7A through 7D are reference diagrams for explaining a method, performed by an ultrasound diagnosis apparatus, of generating and outputting a Doppler sound while operating in an M-mode, according to an embodiment.

Referring to FIG. 7A, according to an embodiment, the ultrasound diagnosis apparatus 100 may operate in an M-mode.

For example, the ultrasound diagnosis apparatus 100 may generate a first ultrasound pulse according to an M-mode PRF and transmit a first ultrasound signal corresponding to the first ultrasound pulse to an object to acquire M-mode data with a first period. In this case, the ultrasound diagnosis apparatus 100 may acquire the M-mode data corresponding to an M line set on a B-mode image.

The ultrasound diagnosis apparatus 100 may generate an M-mode image 720 corresponding to the M line 710 based on the acquired M-mode data and display the M-mode image 720 on the display 140.

Referring to FIG. 7B, according to an embodiment, the ultrasound diagnosis apparatus 100 may receive a user input of inputting a Doppler sound key 730 included in a setup menu screen or a Doppler sound button included in a control panel.

Accordingly, as shown in FIG. 7C, the ultrasound diagnosis apparatus 100 may display an element 740 for setting an ROI on an M line.

The ultrasound diagnosis apparatus 100 may set an ROI based on a user input of moving the element 740, and when the ROI is set, acquire Doppler data corresponding to the ROI with a second period.

For example, as described with reference to FIG. 4, the ultrasound diagnosis apparatus 100 may acquire M mode data together with Doppler data by generating a first ultrasound pulse sequence (an M-mode ultrasound pulse sequence) having a first period alternately with a second ultrasound pulse sequence (a PW mode ultrasound pulse sequence) having a second period. In this case, the second period with which the Doppler data is acquired may be determined based on the first period with which the M-mode data is acquired.

The ultrasound diagnosis apparatus 100 may also generate Doppler data corresponding to an interval during which the Doppler data is not acquired by using the acquired Doppler data. For example, when pieces of Doppler data are respectively acquired during second and fourth intervals and Doppler data is not acquired during a third interval, the ultrasound diagnosis apparatus 100 may interpolate unacquired Doppler data corresponding to the third interval by using the pieces of Doppler data acquired during the second and fourth intervals.

Accordingly, the ultrasound diagnosis apparatus 100 may generate a Doppler sound by using the acquired Doppler data and the interpolated Doppler data and output the Doppler sound.

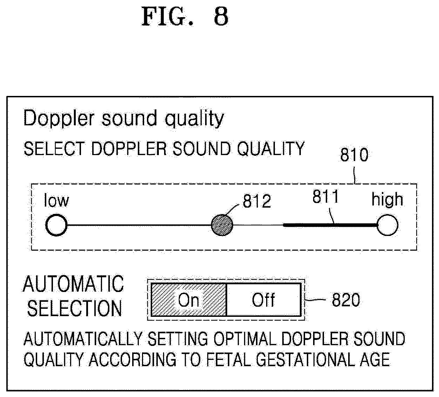

FIG. 8 illustrates an example of a UI capable of adjusting a quality of a Doppler sound output by the ultrasound diagnosis apparatus 100, according to an embodiment.

Referring to FIG. 8, the ultrasound diagnosis apparatus 100 may display a menu screen for adjusting a quality of a Doppler sound.

For example, the menu screen may include a first element 810 that allows a user to adjust a quality of a Doppler sound and a second element 820 that allows the user to set whether to automatically select the quality of the Doppler sound.

The first element 810 may include a first sliding bar 811 containing a first icon 812 that can move to the left or right of the first sliding bar 811.

The user may adjust a Doppler sound quality by moving the first icon 812 to the left or right. For example, a Doppler sound quality may be adjusted to decrease as the first icon 812 is moved to the left and increase as the first icon 812 is moved to the right.

Furthermore, the first sliding bar 811 may be indicated in first through third colors. For example, a part of the first sliding bar 811 from its left end to a first point where the first icon 812 is positioned may be shown in the first color while a part of the first sliding bar 811 from the first point to a second point may be shown in the second color. In this case, the second point may be a point representing a quality of a Doppler sound that can be output when an output of an ultrasound signal corresponding to a PW mode pulse and being applied to an object is set to a maximum within a safe range. The ultrasound diagnosis apparatus 100 may provide the user with a guideline for setting a quality of a Doppler sound by indicating the second point on the first sliding bar 811.

Furthermore, a part of the first sliding bar 811 from the second point to its right end may be shown in the third color.

In addition, the second element 820 may include an On icon and an Off icon, and when the user selects the On icon, the ultrasound diagnosis apparatus 100 may automatically set a quality of a Doppler sound to an optimal quality. However, embodiments are not limited thereto.

According to an embodiment, when the object is a fetus, a Doppler sound quality may be set according to a gestational age of the fetus.

For example, when information about a patient (e.g., information about a pregnant woman or the like) is received, the ultrasound diagnosis apparatus 100 may acquire information about a fetus corresponding to the received information about the patient. The information about the fetus may include a fetal gestational age but is not limited thereto.

According to an embodiment, when a fetal gestational age is less than 12 weeks in the early stage of pregnancy), the ultrasound diagnosis apparatus 100 may set a Doppler sound quality to a first value. When a fetal gestational age is 12 weeks or more (in the middle and late stages of pregnancy), the ultrasound diagnosis apparatus 100 may set a Doppler sound quality to a second value that is greater than the first value. In this case, information about a fetal gestational age may be acquired based on at least one of B-mode data, M-mode data, and Doppler data with respect to a fetus. Furthermore, the information about the fetal gestational age may include at least one of the fetal gestational age and biometric parameters for the fetal gestational age, i.e., a head circumference, an abdominal circumference, a femur length, an abdominal thickness, a transverse trunk diameter, and a crown rump length. However, embodiments are not limited thereto.

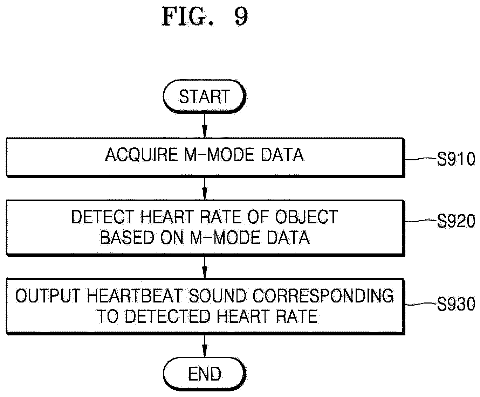

FIG. 9 is a flowchart of a method of operating the ultrasound diagnosis apparatus 100, according to an embodiment.

According to an embodiment, the ultrasound diagnosis apparatus 100 may transmit ultrasound signals to an object and acquire ultrasound data based on echo signals received from the object. For example, the ultrasound diagnosis apparatus 100 may acquire B-mode data with respect to the object based on echo signals. The ultrasound diagnosis apparatus 100 may then generate a B-mode ultrasound image based on the B-mode data and display the generated B-mode ultrasound image. The ultrasound diagnosis apparatus 100 may extract B-mode components from ultrasound data and generate a B-mode ultrasound image indicating signal intensities as brightness values based on the extracted B-mode components.

Furthermore, the ultrasound diagnosis apparatus 100 may set one of a plurality of scan lines included in the B-mode ultrasound image as an M line. A region located on the M line may also be set as an ROI. Furthermore, when the object is a fetus, the fetus's heart may be set as an ROI.

The ultrasound diagnosis apparatus 100 may acquire M-mode data with respect to an object corresponding to an M line (S910). Furthermore, the ultrasound diagnosis apparatus 100 may generate an M-mode image based on the M-mode data and display the M-mode image.

The ultrasound diagnosis apparatus 100 may detect a heart rate of the object (e.g., a fetus) based on period information of the M-mode data (S920).

The ultrasound diagnosis apparatus 100 may output a heartbeat sound corresponding to the detected heart rate (S930).

For example, the ultrasound diagnosis apparatus 100 may prestore a heartbeat sound corresponding to a heart rate. For example, the ultrasound diagnosis apparatus 100 may store a first heartbeat sound corresponding to a heart rate greater than or equal to 120 but less than140 beats per minute (bpm), a second heartbeat sound corresponding to a heart rate greater than or equal to 140 but less than 160 bpm, and a third heartbeat sound corresponding to a heart rate greater than or equal to 160 but less than 180 bpm.

For example, when a heart rate detected based on M-mode data is 150 bpm, the ultrasound diagnosis apparatus 100 may modulate the second heartbeat sound according to 150 bpm (the detected heart rate) and output a resulting sound. However, embodiments are not limited thereto.

Furthermore, the ultrasound diagnosis apparatus 100 may output a heartbeat sound corresponding to a heart rate by further taking into account information about a fetal gestational age.

According to an embodiment, the ultrasound diagnosis apparatus 100 may acquire information about a fetal gestational age based on B-mode data or M-mode data. Information about a fetal gestational age may include at least one of the fetal gestational age and biometric parameters for the fetal gestational age, i.e., a head circumference, an abdominal circumference, a femur length, an abdominal thickness, a transverse trunk diameter, and a crown rump length.

Furthermore, the ultrasound diagnosis apparatus 100 may prestore a standard heartbeat sound for each fetal gestational age. Accordingly, the ultrasound diagnosis apparatus 100 may modulate a standard heartbeat sound corresponding to the acquired information about a fetal gestational age according to a detected heart rate and output a resulting sound.

For example, when a fetal gestational age is 10 weeks and a detected heart rate is 170 bpm, the ultrasound diagnosis apparatus 100 may modulate a standard heartbeat sound corresponding to 10 weeks of gestational age according to the heart rate of 170 bpm and output a resulting sound.

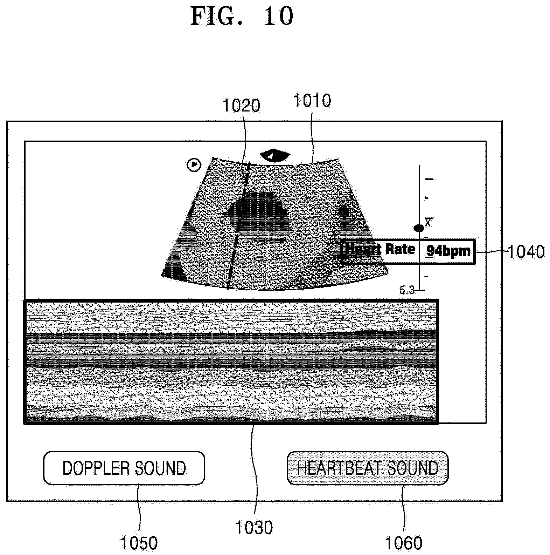

FIG. 10 illustrates an example of a screen displayed by the ultrasound diagnosis apparatus 100, according to an embodiment.

Referring to FIG. 10, the ultrasound diagnosis apparatus 100 may generate a B-mode image 1010 based on B-mode data and display the B-mode image 1010 on the display 140.

The ultrasound diagnosis apparatus 100 may display an M line 1020 superimposed on one of a plurality of scan lines in the B-mode image 1010 and change a position of the M line 1020 based on a user input. The ultrasound diagnosis apparatus 100 may generate an M-mode image 1030 based on M-mode data corresponding to the M line 1020 and display the M-mode image 1030 on the display 140.

Furthermore, the ultrasound diagnosis apparatus 100 may detect a heart rate of an object (e.g., a fetus) and display a detected heart rate 1040.

The ultrasound diagnosis apparatus 100 may provide a UI capable of selecting whether to output a Doppler sound or a heartbeat sound. For example, the Doppler sound may be acquired based on actual Doppler data. The heartbeat sound may be a prestored heartbeat sound corresponding to a heart rate detected in the M-mode data.

When a user selects a Doppler sound icon 1050, the ultrasound diagnosis apparatus 100 may display a screen shown in FIG. 7C. For example, the ultrasound diagnosis apparatus 100 may display an element for setting an ROI on an M- line and acquire Doppler data corresponding to the ROI. Furthermore, the ultrasound diagnosis apparatus 100 may generate a Doppler sound based on the acquired Doppler data and output the Doppler sound.

Otherwise, when the user selects a heartbeat sound icon 1060, the ultrasound diagnosis apparatus 100 may output a heartbeat sound corresponding to a detected heart rate instead of acquiring Doppler data. Detailed descriptions thereof are already provided above with respect to FIG. 9 and thus will not be repeated below.

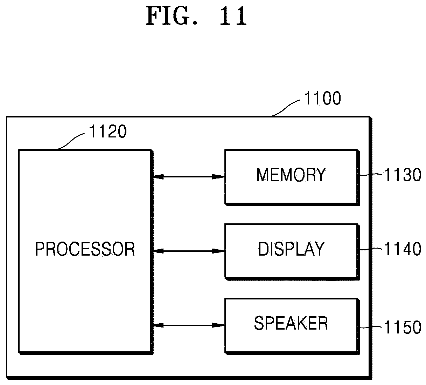

FIG. 11 is a block diagram of a configuration of an ultrasound diagnosis apparatus 1100 according to an embodiment.

Referring to FIG. 11, the ultrasound diagnosis apparatus 1100 according to the embodiment may include a processor 1120, a memory 1130, a display 1140, and a speaker 1150.

The processor 1120 of FIG. 11 may correspond to at least one or a combination of the ultrasound transceiver 110, the controller 120, and the image processor 130 described with reference to FIG. 1, and the display 1140 may correspond to the display 140 of FIG. 1. Furthermore, according to embodiments, some of the components of the ultrasound diagnosis apparatus 100 may be included in the ultrasound diagnosis apparatus 1100 of FIG. 11.

According to an embodiment, the processor 1120 may control all operations of the ultrasound diagnosis apparatus 1100. According to an embodiment, the processor 1120 may execute one or more programs stored in the memory 1130.

According to an embodiment, the memory 1130 may store various data, programs, or applications for driving and controlling the ultrasound diagnosis apparatus 1100. A program stored in the memory 1130 may include one or more instructions. The program (one or more instructions) or application stored in the memory 1130 may be executed by the processor 1120.

According to an embodiment, the processor 1120 may acquire M-mode data with a first period and Doppler data with a second period. In this case, a period of repetition of an M-mode data acquisition interval during which M-mode data is acquired may be the first period (T1 of FIG. 4). Furthermore, a period of repetition of a Doppler data acquisition interval during which Doppler data is acquired may be the second period (T2 of FIG. 4). The processor 1120 may determine the second period T2 based on the first period T1.

With the first period, the processor 1120 may control a probe to transmit a first ultrasound signal corresponding to a first ultrasound pulse to an object and acquire M-mode ultrasound data based on a first echo signal corresponding to the first ultrasound signal. Furthermore, with the second period, the processor 1120 may control the probe to transmit a second ultrasound signal corresponding to a second ultrasound pulse and acquire Doppler data based on a second echo signal corresponding to the second ultrasound signal.

The processor 1120 may generate an M-mode image based on M-mode data. The processor 1120 may interpolate, based on acquired Doppler data, unacquired Doppler data corresponding to an interval during which Doppler data is not acquired. Furthermore, the processor 1120 may generate a Doppler sound based on the acquired Doppler data and the interpolated Doppler data.

The processor 1120 may adjust a quality of a Doppler sound based on a user input and automatically adjust the quality of the Doppler sound based on information of the object. Furthermore, the processor 1120 may adjust the quality of the Doppler sound by adjusting a second period with which Doppler data is acquired.

In addition, the processor 1120 may detect a heart rate of the object based on an M-mode image and determine a heartbeat sound corresponding to the detected heart rate.

According to an embodiment, the display 1140 may display an operating state of the ultrasound diagnosis apparatus 1100, an ultrasound image, a UI, etc. The display 1140 may include one or more display panels according to embodiments and may be formed as a touch screen.

According to an embodiment, the display 1140 may display an M-mode image and a B-mode image of the object together on a single screen. The display 1140 may also display an element for setting an M line and an ROI in the B-mode image.

According to an embodiment, the display 1140 may display a UI for adjusting a quality of a Doppler sound.

According to an embodiment, the speaker 1150 may output a Doppler sound acquired based on Doppler data or a heartbeat sound corresponding to a detected heart rate.

According to an embodiment, a Doppler data acquisition period may be determined according to a M--mode data acquisition period, thereby allowing use of an M-mode in combination with a Doppler mode and safe examination of a fetal heart during early pregnancy.

Furthermore, a Doppler sound of a fetal heart may be provided together with an M-mode image through minimal use of a Doppler mode.

Block diagrams of the ultrasound diagnosis apparatuses 100 and 1100 of FIGS. 1 and 11 may be provided for illustration of embodiments. Each of the components in the block diagram may be integrated, added, or omitted according to the specification of the ultrasound diagnosis apparatus 100 or 1100 that is actually implemented. In other words, two or more components may be combined into a single component, or a single component may be split into two or more components if necessary. Functions performed in each block are intended to describe embodiments, and a specific operation or apparatus related to the functions does not limit the scope of the disclosure.

Methods of operating an ultrasound diagnosis apparatus may be implemented in the form of program instructions that may be performed by various types of computers and may be recorded on non-transitory computer-readable recording media. The non-transitory computer-readable recording media may include program instructions, data files, data structures, etc. either alone or in combination. The program instructions recorded on the non-transitory computer-readable recording media may be designed and configured specially for the disclosure or may be known to and be usable by those skilled in the art of computer software. Examples of the non-transitory computer-readable recording media include magnetic media such as hard disks, floppy disks, and magnetic tape, optical media such as compact disk read-only memory (CD-ROM) and digital versatile disks (DVDs), magneto-optical media such as floptical disks, and hardware devices that are specially configured to store and perform program instructions, such as ROM, random access memory (RAM), flash memory, and the like. Examples of program instructions include not only machine code such as that created by a compiler but also higher level language code that may be executed by a computer using an interpreter or the like.

Furthermore, ultrasound diagnosis apparatuses and methods of operating an ultrasound diagnosis apparatus according to embodiments of the disclosure may be included in a computer program product when provided. The computer program product may be traded, as a commodity, between a seller and a buyer.

The computer program product may include a software program and a computer-readable storage medium having stored thereon the software program. For example, the computer program product may include a product (e.g. a downloadable application) in the form of a software program electronically distributed by a manufacturer of an electronic device or through an electronic market (e.g., Google Play Store ^{™} and App Store ^{™}). For such electronic distribution, at least a part of the software program may be stored on the computer-readable storage medium or may be temporarily generated. In this case, the computer-readable storage medium may be a storage medium of a server of the manufacturer, a server of the electronic market, or a relay server for temporarily storing the software program.

In a system consisting of a server and a client device, the computer program product may include a storage medium of the server or client device. Alternatively, in a case where a third device (e.g., a smartphone) is connected to the server or client device through a communication network, the computer program product may include a storage medium of the third device. Alternatively, the computer program product may include a software program that is transmitted from the server to the client device or the third device or that is transmitted from the third device to the client device.

In this case, one of the server, the client device, and the third device may execute the computer program product to perform the methods according to embodiments of the disclosure. Alternatively, two or more of the server, the client device, and the third device may execute the computer program product to perform the methods according to the embodiments in a distributed manner.

For example, the server (e.g., a cloud server, an artificial intelligence (AI) server, or the like) may run the computer program product stored therein to control the client device communicating with the server to perform the methods according to the embodiments.

While one or more embodiments have been particularly described with reference to the figures, it will be understood by those of ordinary skill in the art that the embodiments are not to be construed as limiting the scope of the present disclosure.

## Claims

1. An ultrasound diagnosis apparatus comprising:
a memory (1130) storing one or more instructions;
a processor (1120) configured to execute the one or more instructions to acquire, based on echo signals received from an object, motion (M) mode data with respect to the object with a first period and Doppler data with respect to the object with a second period, generate an M mode image based on the M mode data, generate a Doppler sound based on the Doppler data, control a display to display the M mode image, and control a speaker to output the Doppler sound; and
a probe configured to transmit an ultrasound signal to the object and receive the echo signals from the object,
wherein the processor (1120) is further configured to execute the one or more instructions to:
control the probe to transmit with the first period a first ultrasound signal corresponding to a first ultrasound pulse according to an M-mode pulse repetition frequency to the object and acquire the M mode data based on a first echo signal corresponding to the first ultrasound signal; and
control the probe to transmit with the second period a second ultrasound signal corresponding to a sequence of second ultrasound pulses according to a pulsed wave mode pulse repetition frequency to the object and acquire the Doppler data based on a second echo signal corresponding to the second ultrasound signal,
wherein the second period is determined based on the first period,
wherein the Doppler data is acquired during a first time interval and a third time interval,
wherein the difference between the beginning of the first time interval and the beginning of the third time interval is the second period,
wherein the Doppler data is unacquired during a second time interval, which is the remaining interval between the first time interval and the third time interval, and
wherein the processor interpolates unacquired Doppler data corresponding to the second time interval during which the Doppler data is not acquired, based on the acquired Doppler data corresponding to the first time interval and the third time interval.

2. The ultrasound diagnosis apparatus of claim 1, wherein the processor is further configured to execute the one or more instructions to:
acquire brightness (B) mode data with respect to the object based on the echo signals, generate a B mode image based on the B mode data, and control the display to display the B-mode image; and
set an M line and a region of interest in the B mode image based on a user input, wherein the M mode data is data corresponding to the M line, and the Doppler data is data corresponding to the region of interest.

3. The ultrasound diagnosis apparatus of claim 2, wherein the display displays the B mode image of the object and the M mode image corresponding to the M line on a single screen,
wherein the speaker is configured to output the Doppler sound corresponding to the region of interest simultaneously with the displaying of the B mode image and the M mode image.

4. The ultrasound diagnosis apparatus of claim 1, wherein the processor is further configured to execute the one or more instructions to acquire brightness (B) mode data with respect to the object during a fourth time interval that is between the first and third time intervals, generate a B mode image based on the B mode data, and control the display to display the B mode image.

5. The ultrasound diagnosis apparatus of claim 1, wherein the processor is further configured to execute the one or more instructions to control the display to display a user interface capable of adjusting a quality of the Doppler sound and adjust the quality of the Doppler sound based on a user input via the user interface.

6. The ultrasound diagnosis apparatus of claim 1, wherein the processor is further configured to execute the one or more instructions to automatically adjust a quality of the Doppler sound based on information about the object.

7. The ultrasound diagnosis apparatus of claim 1, wherein the processor is further configured to execute the one or more instructions to adjust a quality of the Doppler sound by adjusting the second period with which the Doppler data is acquired.

8. The ultrasound diagnosis apparatus of claim 1, wherein the processor is further configured to execute the one or more instructions to detect a heart rate of the object based on the M mode image and control the display to display the heart rate.

9. The ultrasound diagnosis apparatus of claim 8, wherein the processor is further configured to execute the one or more instructions to determine whether there is an abnormality in the heart rate and control the display to display a result of the determining.

10. The ultrasound diagnosis apparatus of claim 8, wherein the processor is further configured to execute the one or more instructions to determine a heartbeat sound corresponding to the heart rate of the object and control the speaker to output the heartbeat sound.

11. A method of operating an ultrasound diagnosis apparatus, the method comprising:
acquiring, based on echo signals received from an object, motion (M) mode data with respect to the object with a first period and Doppler data with respect to the object with a second period;
generating an M mode image based on the M mode data;
generating a Doppler sound based on the Doppler data;
displaying the M mode image;
outputting the Doppler sound; and
transmitting ultrasound signals to the object and receiving the echo signals from the object,
wherein the second period is determined based on the first period,
wherein the acquiring of the M mode data and the Doppler data comprises:
transmitting a first ultrasound signal corresponding to a first ultrasound pulse according to an M-mode pulse repetition frequency to the object with the first period;
acquiring the M mode data based on a first echo signal corresponding to the first ultrasound signal;
transmitting a second ultrasound signal corresponding to a sequence of second ultrasound pulses according to a pulsed wave mode pulse repetition frequency to the object with the second period; and
acquiring the Doppler data based on a second echo signal corresponding to the second ultrasound signal,
wherein the Doppler data is acquired during a first time interval and a third time interval,
wherein the difference between the beginning of the first time interval and the beginning of the third time interval is the second period,
wherein the Doppler data is unacquired during a second time interval, which is the remaining interval between the first time interval and the third time interval, and
wherein a processor interpolates unacquired Doppler data corresponding to the second time interval during which the Doppler data is not acquired, based on the acquired Doppler data corresponding to the first time interval and the third time interval.

12. The method of claim 11, further comprising:
displaying a brightness (B) mode image of the object; and
setting an M line and a region of interest in the B mode image based on a user input,
wherein the M mode data is data corresponding to the M line, and the Doppler data is data corresponding to the region of interest.

## Patentansprüche

1. Ultraschalldiagnosevorrichtung, die Folgendes umfasst:
einen Speicher (1130), in dem eine oder mehrere Anweisungen gespeichert sind;
einen Prozessor (1120), der so konfiguriert ist, dass er die eine oder mehreren Anweisungen ausführt, um basierend auf von einem Objekt empfangenen Echosignalen Bewegungs- (M) -Modusdaten in Bezug auf das Objekt mit einer ersten Periode und Doppler-Daten in Bezug auf das Objekt mit einer zweiten Periode zu erfassen, ein M-Modus-Bild basierend auf den M-Modus-Daten zu erzeugen, einen Dopplerton basierend auf den Doppler-Daten zu erzeugen, eine Anzeige zum Anzeigen des M-Modus-Bildes zu steuern und einen Lautsprecher zum Ausgeben des Dopplertons zu steuern; und
eine Sonde, die so konfiguriert ist, dass sie ein Ultraschallsignal an das Objekt überträgt und die Echosignale von dem Objekt empfängt,
wobei der Prozessor (1120) ferner so konfiguriert ist, dass er die eine oder mehreren Anweisungen zu Folgendem ausführt:
Steuern der Sonde zum Übertragen eines einem ersten Ultraschallimpuls entsprechenden ersten Ultraschallsignals mit der ersten Periode gemäß einer M-Modus-Impulswiederholungsfrequenz an das Objekt und Erfassen der M-Modus-Daten basierend auf einem dem ersten Ultraschallsignal entsprechenden ersten Echosignal; und
Steuern der Sonde zum Übertragen eines einer Sequenz von zweiten Ultraschallimpulsen entsprechenden zweiten Ultraschallsignals mit der zweiten Periode gemäß einer Impulswiederholungsfrequenz im gepulsten Wellenmodus an das Objekt und Erfassen der Doppler-Daten basierend auf einem dem zweiten Ultraschallsignal entsprechenden zweiten Echosignal,
wobei die zweite Periode basierend auf der ersten Periode bestimmt wird,
wobei die Doppler-Daten während eines ersten Zeitintervalls und eines dritten Zeitintervalls erfasst werden,
wobei die Differenz zwischen dem Beginn des ersten Zeitintervalls und dem Beginn des dritten Zeitintervalls die zweite Periode ist,
wobei in einem zweiten Zeitintervall, das das verbleibende Intervall zwischen dem ersten Zeitintervall und dem dritten Zeitintervall ist, keine Doppler-Daten erfasst werden, und
wobei der Prozessor basierend auf den erfassten Doppler-Daten, die dem ersten Zeitintervall und dem dritten Zeitintervall entsprechen, nicht erfasste Doppler-Daten interpoliert, die dem zweiten Zeitintervall entsprechen, in dem keine Doppler-Daten erfasst wurden.

2. Ultraschalldiagnosevorrichtung nach Anspruch 1, wobei der Prozessor ferner so konfiguriert ist, dass er die eine oder mehreren Anweisungen zu Folgendem ausführt:
Erfassen von Helligkeits- (B) -Modus-Daten in Bezug auf das Objekt basierend auf den Echosignalen, Erzeugen eines B-Modus-Bildes basierend auf den B-Modus-Daten, und Steuern der Anzeige, um das B-Modus-Bild anzuzeigen; und
Einstellen einer M-Linie und eines Bereichs von Interesse in dem B-Modus-Bild basierend auf einer Benutzereingabe, wobei die M-Modus-Daten Daten sind, die der M-Linie entsprechen, und die Doppler-Daten Daten sind, die dem Bereich von Interesse entsprechen.

3. Ultraschalldiagnosevorrichtung nach Anspruch 2, wobei die Anzeige das B-Modus-Bild des Objekts und das der M-Linie entsprechende M-Modus-Bild auf einem einzigen Bildschirm anzeigt,
wobei der Lautsprecher so konfiguriert ist, dass er den dem Bereich von Interesse entsprechenden Dopplerton gleichzeitig mit der Anzeige des B-Modus-Bildes und des M-Modus-Bildes ausgibt.

4. Ultraschalldiagnosevorrichtung nach Anspruch 1, wobei der Prozessor ferner so konfiguriert ist, dass er die eine oder mehreren Anweisungen ausführt, um während eines vierten Zeitintervalls, das zwischen dem ersten und dem zweiten Zeitintervall liegt, Helligkeits- (B) -Modus-Daten in Bezug auf das Objekt zu erfassen, ein B-Modus-Bild basierend auf den B-Modus-Daten zu erzeugen und die Anzeige zu steuern, um das B-Modus-Bild anzuzeigen.

5. Ultraschalldiagnosevorrichtung nach Anspruch 1, wobei der Prozessor ferner so konfiguriert ist, dass er die eine oder mehreren Anweisungen ausführt, um die Anzeige zum Anzeigen einer Benutzerschnittstelle zu steuern, die in der Lage ist, eine Qualität des Dopplertons einzustellen und die Qualität des Dopplertons basierend auf einer Benutzereingabe über die Benutzerschnittstelle einzustellen.

6. Ultraschalldiagnosevorrichtung nach Anspruch 1, wobei der Prozessor ferner so konfiguriert ist, dass er die eine oder mehreren Anweisungen ausführt, um eine Qualität des Dopplertons basierend auf Informationen über das Objekt automatisch einzustellen.

7. Ultraschalldiagnosevorrichtung nach Anspruch 1, wobei der Prozessor ferner so konfiguriert ist, dass er die eine oder mehreren Anweisungen ausführt, um eine Qualität des Dopplertons einzustellen, indem er die zweite Periode anpasst, mit der die Doppler-Daten erfasst werden.

8. Ultraschalldiagnosevorrichtung nach Anspruch 1, wobei der Prozessor ferner so konfiguriert ist, dass er die eine oder mehreren Anweisungen ausführt, um eine Herzfrequenz des Objekts basierend auf dem M-Modus-Bild zu erfassen und die Anzeige zu steuern, um die Herzfrequenz anzuzeigen.

9. Ultraschalldiagnosevorrichtung nach Anspruch 8, wobei der Prozessor ferner so konfiguriert ist, dass er die eine oder mehreren Anweisungen ausführt, um zu bestimmen, ob eine Anomalie in der Herzfrequenz vorliegt, und die Anzeige steuert, um ein Ergebnis des Bestimmens anzuzeigen.

10. Ultraschalldiagnosevorrichtung nach Anspruch 8, wobei der Prozessor ferner so konfiguriert ist, dass er die eine oder mehreren Anweisungen ausführt, um ein Herzschlaggeräusch zu bestimmen, das der Herzfrequenz des Objekts entspricht, und den Lautsprecher so steuert, dass er das Herzschlaggeräusch ausgibt.

11. Verfahren zum Betrieb einer Ultraschalldiagnosevorrichtung, wobei das Verfahren Folgendes umfasst:
basierend auf von einem Objekt empfangenen Echosignalen, Erfassen von Bewegungs- (M) -Modusdaten in Bezug auf das Objekt mit einer ersten Periode und Doppler-Daten in Bezug auf das Objekt mit einer zweiten Periode;
Erzeugen eines M-Modus-Bildes basierend auf den M-Modus-Daten;
Erzeugen eines Dopplertons basierend auf den Doppler-Daten;
Anzeigen des M-Modus-Bildes;
Ausgeben des Dopplertons; und
Übertragen von Ultraschallsignalen an das Objekt und Empfangen der Echosignale von dem Objekt,
wobei die zweite Periode basierend auf der ersten Periode bestimmt wird,
wobei das Erfassen der M-Modus-Daten und der Doppler-Daten Folgendes umfasst:
Übertragen eines ersten Ultraschallsignals, das einem ersten Ultraschallimpuls entspricht, gemäß einer M-Modus-Impulswiederholungsfrequenz an das Objekt mit der ersten Periode;
Erfassen der M-Modus-Daten basierend auf einem ersten Echosignal, das dem ersten Ultraschallsignal entspricht;
Übertragen eines zweiten Ultraschallsignals, das einer Sequenz von zweiten Ultraschallimpulsen entspricht, gemäß einer Impulswiederholungsfrequenz im gepulsten Wellenmodus an das Objekt mit der zweiten Periode; und
Erfassen der Doppler-Daten basierend auf einem zweiten Echosignal, das dem zweiten Ultraschallsignal entspricht,
wobei die Doppler-Daten in einem ersten Zeitintervall und einem dritten Zeitintervall erfasst werden,
wobei die Differenz zwischen dem Beginn des ersten Zeitintervalls und dem Beginn des dritten Zeitintervalls die zweite Periode ist,
wobei in einem zweiten Zeitintervall, das das verbleibende Intervall zwischen dem ersten Zeitintervall und dem dritten Zeitintervall ist, keine Doppler-Daten erfasst werden, und
wobei der Prozessor basierend auf den erfassten Doppler-Daten, die dem ersten Zeitintervall und dem dritten Zeitintervall entsprechen, nicht erfasste Doppler-Daten interpoliert, die dem zweiten Zeitintervall entsprechen, in dem keine Doppler-Daten erfasst wurden.

12. Verfahren nach Anspruch 11, das ferner Folgendes umfasst:
Anzeigen eines Helligkeits- (B) -Modus-Bildes des Objekts; und
Einstellen einer M-Linie und eines Bereichs von Interesse in dem B-Modus-Bild basierend auf einer Benutzereingabe,
wobei die M-Modus-Daten Daten sind, die der M-Linie entsprechen, und die Doppler-Daten Daten sind, die dem Bereich von Interesse entsprechen.

## Revendications

1. Dispositif d'échographie diagnostique comprenant :
une mémoire (1130) dans laquelle sont stockées une ou plusieurs instructions,
un processeur (1120) configuré pour exécuter la ou les instructions de façon à acquérir, compte tenu de signaux d'écho reçus en provenance d'un objet, des données en mode mouvement (M) relatives à l'objet avec une première période et des données Doppler relatives à l'objet avec une deuxième période, générer une image en mode M compte tenu des données en mode M, générer un son Doppler compte tenu des données Doppler, commander un écran de manière qu'il affiche l'image en mode M et commander un haut-parleur de manière qu'il produise le son Doppler, et
une sonde configurée pour transmettre un signal ultrasonore à l'objet et recevoir les signaux d'écho en provenant ;
ledit processeur (1120) étant en outre configuré pour exécuter la ou les instructions de façon à:
commander la sonde de manière qu'elle transmette, avec la première période, un premier signal ultrasonore correspondant à une première impulsion ultrasonore selon une fréquence de répétition d'impulsions en mode M à destination de l'objet, et qu'elle acquiert les données en mode M compte tenu d'un premier signal d'écho correspondant au premier signal ultrasonore ; et
commander la sonde de manière qu'elle transmette, avec la deuxième période, un deuxième signal ultrasonore correspondant à une séquence de deuxièmes impulsions ultrasonores selon une fréquence de répétition d'impulsions en mode onde pulsée à destination de l'objet, et qu'elle acquiert les données Doppler compte tenu d'un deuxième signal d'écho correspondant au deuxième signal ultrasonore ;
ladite deuxième période étant déterminée compte tenu de ladite première période,
lesdites données Doppler étant acquises pendant un premier intervalle de temps et un troisième intervalle de temps,
la différence entre le début du premier intervalle de temps et le début du troisième intervalle de temps constituant la deuxième période,
lesdites données Doppler n'étant pas acquises pendant un deuxième intervalle de temps, qui consiste en l'intervalle restant entre le premier intervalle de temps et le troisième intervalle de temps, et
ledit processeur effectuant une interpolation concernant les données Doppler non acquises correspondant au deuxième intervalle de temps pendant lequel les données Doppler ne sont pas acquises, compte tenu des données Doppler acquises correspondant au premier intervalle de temps et au troisième intervalle de temps.

2. Dispositif d'échographie diagnostique selon la revendication 1, dans lequel le processeur est en outre configuré pour exécuter la ou les instructions de façon à :
acquérir des données en mode luminosité (B) relatives à l'objet compte tenu des signaux d'écho, générer une image en mode B compte tenu des données en mode B, et commander l'écran de manière qu'il affiche l'image en mode B, et
définir une ligne M et une région d'intérêt dans l'image en mode B en fonction d'une entrée utilisateur, les données en mode M consistant en des données correspondant à la ligne M et les données Doppler consistant en des données correspondant à la région d'intérêt.

3. Dispositif d'échographie diagnostique selon la revendication 2, dans lequel l'écran affiche l'image en mode B de l'objet et l'image en mode M correspondant à la ligne M sur un même visuel,
ledit haut-parleur étant configuré pour produire le son Doppler correspondant à la région d'intérêt simultanément à l'affichage de l'image en mode B et de l'image en mode M.

4. Dispositif d'échographie diagnostique selon la revendication 1, dans lequel le processeur est en outre configuré pour exécuter la ou les instructions de façon à acquérir des données en mode luminosité (B) relatives à l'objet pendant un quatrième intervalle de temps qui se situe entre les premier et deuxième intervalles de temps, générer une image en mode B compte tenu des données en mode B et commander l'écran de manière qu'il affiche l'image en mode B.

5. Dispositif d'échographie diagnostique selon la revendication 1, dans lequel le processeur est en outre configuré pour exécuter la ou les instructions de façon à commander l'écran de manière qu'il affiche une interface utilisateur capable de régler la qualité du son Doppler, et de façon à ajuster la qualité du son Doppler en fonction d'une entrée utilisateur effectuée par l'intermédiaire de l'interface utilisateur.

6. Dispositif d'échographie diagnostique selon la revendication 1, dans lequel le processeur est en outre configuré pour exécuter la ou les instructions de façon à ajuster automatiquement la qualité du son Doppler compte tenu d'informations concernant l'objet.

7. Dispositif d'échographie diagnostique selon la revendication 1, dans lequel le processeur est en outre configuré pour exécuter la ou les instructions de façon à ajuster la qualité du son Doppler en ajustant la deuxième période avec laquelle les données Doppler sont acquises.

8. Dispositif d'échographie diagnostique selon la revendication 1, dans lequel le processeur est en outre configuré pour exécuter la ou les instructions de façon à détecter la fréquence cardiaque de l'objet compte tenu de l'image en mode M et à commander l'écran de manière qu'il affiche la fréquence cardiaque.

9. Dispositif d'échographie diagnostique selon la revendication 8, dans lequel le processeur est en outre configuré pour exécuter la ou les instructions de façon à déterminer s'il existe une anomalie dans la fréquence cardiaque et à commander l'écran de manière qu'il affiche le résultat de la détermination.

10. Dispositif d'échographie diagnostique selon la revendication 8, dans lequel le processeur est en outre configuré pour exécuter la ou les instructions de façon à déterminer un son de battement cardiaque correspondant à la fréquence cardiaque de l'objet et à commander le haut-parleur de manière qu'il produise le son de battement cardiaque.

11. Procédé d'exploitation d'un dispositif d'échographie diagnostique, le procédé comprenant :
l'acquisition, compte tenu de signaux d'écho reçus en provenance d'un objet, de données en mode mouvement (M) relatives à l'objet avec une première période et de données Doppler relatives à l'objet avec une deuxième période,
la génération d'une image en mode M compte tenu des données en mode M,
la génération d'un son Doppler compte tenu des données Doppler,
l'affichage de l'image en mode M,
la production du son Doppler, et
la transmission de signaux ultrasonores à destination de l'objet et la réception des signaux d'écho en provenant,
ladite deuxième période étant déterminée compte tenu de ladite première période,
ladite acquisition des données en mode M et des données Doppler comprenant :
la transmission d'un premier signal ultrasonore correspondant à une première impulsion ultrasonore selon une fréquence de répétition d'impulsions en mode M à destination de l'objet avec la première période,
l'acquisition des données en mode M compte tenu d'un premier signal d'écho correspondant au premier signal ultrasonore,
la transmission d'un deuxième signal ultrasonore correspondant à une séquence de deuxièmes impulsions ultrasonores selon une fréquence de répétition d'impulsions en mode onde pulsée à destination de l'objet avec la deuxième période, et
l'acquisition des données Doppler compte tenu d'un deuxième signal d'écho correspondant au deuxième signal ultrasonore ;
lesdites données Doppler étant acquises pendant un premier intervalle de temps et un troisième intervalle de temps,
la différence entre le début du premier intervalle de temps et le début du troisième intervalle de temps constituant la deuxième période,
lesdites données Doppler n'étant pas acquises pendant un deuxième intervalle de temps, qui consiste en l'intervalle restant entre le premier intervalle de temps et le troisième intervalle de temps, et
ledit processeur effectuant une interpolation concernant les données Doppler non acquises correspondant au deuxième intervalle de temps pendant lequel les données Doppler ne sont pas acquises, compte tenu des données Doppler acquises correspondant au premier intervalle de temps et au troisième intervalle de temps.

12. Procédé selon la revendication 11, comprenant en outre :
l'affichage d'une image de l'objet en mode luminosité (B), et
définition d'une ligne M et d'une région d'intérêt dans l'image en mode B en fonction d'une entrée utilisateur,
les données en mode M étant des données correspondant à la ligne M, et les données Doppler étant des données correspondant à la région d'intérêt.
